(19) Europäisches Patentamt
European Patent Office
Office européen des brevets

(11) Veröffentlichungsnummer: **0 209 785 B1**

(12) # EUROPÄISCHE PATENTSCHRIFT

(45) Veröffentlichungstag der Patentschrift: **19.07.89**

(51) Int. Cl.⁴: **C07C 49/04**, C07C 45/54, C07C 11/18, C07C 1/253

(21) Anmeldenummer: **86109258.3**

(22) Anmeldetag: **07.07.86**

(54) **Verfahren zur Herstellung von Methylisopropylketon und Isopren.**

(30) Priorität: **23.07.85 DE 3526247**

(43) Veröffentlichungstag der Anmeldung: **28.01.87 Patentblatt 87/5**

(45) Bekanntmachung des Hinweises auf die Patenterteilung: **19.07.89 Patentblatt 89/29**

(84) Benannte Vertragsstaaten: **BE CH DE FR GB IT LI NL**

(56) Entgegenhaltungen:
**EP-A- 0 080 449**
**EP-A- 0 162 385**
**EP-A- 0 162 387**
**CH-A- 162 145**
**US-A- 4 537 995**

**CHEMICAL ABSTRACTS, Band 98, 1983, Seite 112, Zusammenfassung Nr. 217599k, Columbus, Ohio, US; D.A. BOL'SHAKOV et al.: "Production of isoprene from 2-butenes and synthesis gas via 2-methylbutanal"**

(73) Patentinhaber: **BASF Aktiengesellschaft, Carl-Bosch-Strasse 38, D-6700 Ludwigshafen(DE)**

(72) Erfinder: **Hoelderich, Wolfgang, Dr., Mannheimer Strasse 18c, D-6710 Frankenthal(DE)**
Erfinder: **Merger, Franz, Dr., Max-Slevogt-Strasse 25, D-6710 Frankenthal(DE)**
Erfinder: **Kratzer, Otto, Dr., An der Tuchbleiche 7, D-6712 Bobenheim-Roxheim(DE)**
Erfinder: **Scheidmeir, Walter, Dr., Hardenburgstrasse 41, D-6703 Limburgerhof(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 209 785 B1

**Beschreibung**

In der älteren Anmeldung EP 85105768 (OS 0 162 387) wurde vorgeschlagen, Ketone durch Isomerisierung von Aldehyden bei Temperaturen bis 600°C in Gegenwart von Zeolithen als Katalysatoren herzustellen.

Durch Umsetzung von reinem Pivalinaldehyd (2,2-Dimethylpropanal) wurde hierbei Methylisopropylketon nach Gleichung (1) gebildet.

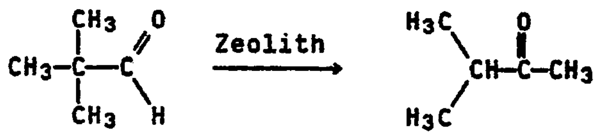


(1)

In der älteren Anmeldung EP 85105766 (OS 0 162 385) wurde weiterhin vorgeschlagen, Diene durch Dehydratisierung von Aldehyden bei höherer Temperatur unter Verwendung von zeolithischen Katalysatoren herzustellen. Isopren wurde durch Umsetzung von Pivalinaldehyd, 2-Methyl- oder 3-Methylbutanal gemäß Gleichung (2) erhalten.

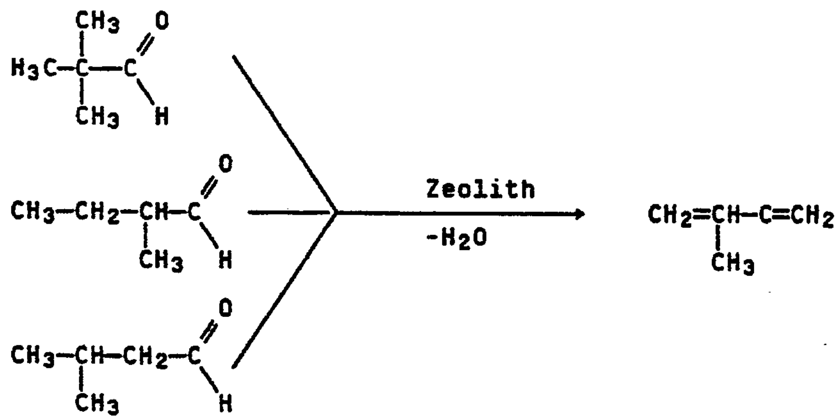


(2)

Da bei technischen Prozessen häufig Produktgemische von iso-Pentanalen anfallen, die zudem noch weitere Komponenten wie n-Alkanale, tertiäre Alkohole, Kohlenwasserstoffe und/oder Wasser enthalten können, bestand die Aufgabe, Methylisopropylketon und Isopren, ausgehend von technischen Reaktionsgemischen herzustellen, ohne daß Nebenreaktionen der verschiedenen Komponenten untereinander auftreten.

Es wurde nun gefunden, daß man Methylisopropylketon und Isopren durch Umsetzung von Pivalinaldehyd an zeolithischen Katalysatoren bei erhöhter Temperatur vorteilhaft herstellt, wenn man für die Umsetzung Hydroformylierungsgemische des Isobutens oder isobutenhaltiger $C_4$-Schnitte verwendet.

Die für das Verfahren in Betracht kommenden Einsatzgemische werden durch Hydroformylierung von Isobuten oder isobutenhaltigen $C_4$-Schnitten unter Katalyse von Carbonylkomplexen von Metallen der 8. Gruppe des Periodensystems bei erhöhtem Druck und erhöhter Temperatur nach üblichen Methoden, z.B. nach W.J. Schneider, Chemiker-Zeitung 96 (1972) 7, 383-387 oder nach J.E. Knap, N.R. Cox und W.R. Privette, Chem.Eng.Progress 62 (1966) 4, 74-78 erhalten. Zur Erzielung hoher Anteile an Methylisopropylketon können die im rohen Reaktionsgemisch der Hydroformylierung neben Pivalinaldehyd vorliegenden Aldehyde wie n-Butyraldehyd, n-Valeraldehyd, 2-Methyl- und 3-Methylbutanal ganz oder teilweise vor der Umsetzung am Zeolithen entfernt werden. Bevorzugt werden als Ausgangsgemische die nach Hydroformylierung des Isobutens und vollständiger oder teilweiser Entfernung des 3-Methylbutanals erhaltenen Produktgemische umgesetzt. Im Reaktionsgemisch gegebenenfalls vorliegendes tert.-Butanol hat keinen Einfluß auf die Umsetzung, es wird mit hoher Selektivität vollständig zu Isobuten gemäß Gleichung (3) dehydratisiert

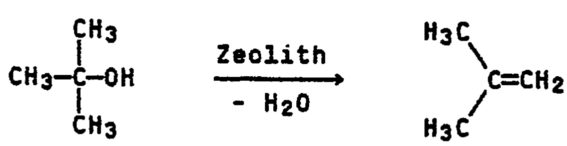


(3)

und wird vorteilhaft vom entstehenden Isopren abgetrennt, um es z.B. für den Hydroformylierungsschritt wiederzuverwenden.

Die in der älteren Anmeldung EP 85105768 vorgeschlagenen Katalysatoren, insbesondere Alumino-, Boro- und Eisensilikatzeolithe vom Pentasiltyp werden vorteilhaft auch für die Umsetzung der beschriebenen rohen Reaktionsgemische, die Pivalinaldehyd in der Regel in einer Menge von 40 bis 95 Gew.% enthalten, verwendet. Überraschenderweise werden keine Nebenreaktionen wie Aldolkondensation oder Oligomerisierung beobachtet. Das in der älteren Anmeldung beschriebene Verfahren ist also nicht auf die Verwendung von Reinsubstanzen als Ausgangskomponenten beschränkt und liefert ausgehend von technischen Produktgemischen hohe Selektivitäten und Umsätze bei hohen Katalysatorstandzeiten. Aufwendiges Abtrennen und Reinigen der Ausgangssubstanzen erübrigt sich somit.

Der Aluminosilikatzeolith wird z.B. aus einer Aluminiumverbindung, vorzugsweise $Al(OH)_3$ oder $Al_2(SO_4)_3$, und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz bei 100 bis 220°C unter autogenem Druck hergestellt. Auch gehören hierzu die isotaktischen Zeolithe nach DE-OS 30 06 471. Die erhaltenen Aluminosilikatzeolithe enthalten je nach Wahl der Einsatzstoffmengen ein $SiO_2/Al_2O_3$-Verhältnis von 10 bis 40.000. Die Aluminosilikatzeolithe lassen sich auch in etherischem Medium, wie Diethylenglykoldimethylether, in alkoholischem Medium, wie Methanol bzw. 1,4-Butandiol oder in Wasser herstellen.

Der Borosilikatzeolith wird z.B. bei 90 bis 200°C unter autogenem Druck synthetisiert, indem man eine Borverbindung, z.B. $H_3BO_3$, mit einer Siliciumverbindung, vorzugsweise mit hochdispersem Siliciumdioxid, in wäßriger Aminlösung insbesondere in 1,6-Hexandiamin- oder 1,3-Propandiamin- oder Triethylentetramin-Lösung mit und ohne Alkali- oder Erdalkalizusatz zur Reaktion bringt. Man kann bei dieser Reaktion anstelle einer wäßrigen Aminlösung eine etherische Lösung, z.B. mit Diethylenglykoldimethylether, oder eine alkoholische Lösung, z.B. mit 1,6-Hexandiol als Lösungsmittel verwenden.

Den Eisensilikatzeolith erhält man z.B. aus einer Eisenverbindung, vorzugsweise $Fe_2(SO_4)_3$ und einer Siliciumverbindung, vorzugsweise aus hochdispersem Siliciumdioxid in wäßriger Aminlösung, insbesondere 1,6-Hexandiamin, mit und ohne Alkali- oder Erdalkalizusatz bei 100 bis 200°C unter autogenem Druck.

Die so hergestellten Alumino-, Boro- und Eisensilikatzeolithe können nach ihrer Isolierung, Trocknung bei 100 bis 160°C, vorzugsweise 110°C und Calcination bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 95:5, bevorzugt 75:25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge bei 110°C/16 h getrocknet und bei 500°C/16 h calciniert. Besonders vorteilhaft lassen sich solche Katalysatoren dadurch herstellen, daß man den isolierten Alumino- bzw. Boro- bzw. Eisensilikatzeolith direkt nach der Trocknung verformt und erstmals nach der Verformung einer Calcination unterwirft. Aus dem zu Strängen verformten Katalysator kann man durch Mahlen und Sieben Wirbelgut mit der Teilchengröße von 0.1 bis 0,5 mm erhalten. Die Alumino-, Boro- und Eisensilikatzeolithe können aber auch in reiner Form, ohne Binder, als Stränge oder Tabletten eingesetzt werden. Diese Verformung erfolgt unter Zusatz von Verstrangungshilfs- bzw. Peptisierungsmitteln wie z.B. Hexaethylcellulose, Stearinsäure, Kartoffelstärke, Ameisensäure, Essigsäure, Oxalsäure, Salpetersäure, Ammoniak, Amine, Silicoester, Graphit oder deren Gemische. Liegt der Zeolith aufgrund der Art seiner Herstellung nicht in der katalytisch bevorzugten aciden H-Form, sondern z.B. in der Na-Form vor, dann kann diese durch Ionenaustausch mit Ammoniumionen und anschließende Calcination oder durch Behandlung mit Säuren vollkommen oder partiell in die gewünschte H-Form übergeführt werden.

Zur Erhöhung der Selektivität, der Standzeit und der Anzahl an Regenerierungen ist es mitunter vorteilhaft, Modifizierungen an den Zeolithen vorzunehmen. Eine geeignete Modifizierung der Katalysatoren besteht z.B. darin, daß man den unverformten oder verformten Zeolithen mit Alkali - sofern nicht schon von der Synthese her die Alkali-Form des Zeolithen vorliegt -mit Erdalkali wie Ca, Mg und Erdmetallen wie B, Ti, ionenaustauschen bzw. imprägnieren kann. Insbesondere ist eine Dotierung der Zeolithe mit Übergangsmetallen, wie Mo, W, Fe, Zn, Cu, mit Edelmetallen, wie Pd und mit seltenen Erdmetallen, wie Ce, La, vorteilhaft.

Praktisch stellt man solche modifizierten Kontakte z.B. so her, daß man den verformten Pentasilzeolithen in einem Steigrohr vorlegt und bei 20 bis 100°C z.B. eine wäßrige oder ammoniakalische Lösung eines Halogenids oder eines Nitrats der voranbeschriebenen Metalle darüberleitet. Ein derartiger Ionenaustausch kann z.B. an der Wasserstoff-, Ammonium- und Alkaliform des Zeolithen vorgenommen werden. Man kann die Metallaufbringung auf den Zeolithen z.B. auch so vornehmen, daß man das zeolithische Material z.B. mit einem Halogenid, einem Nitrat oder einem Oxid der voranbeschriebenen Metalle in wäßriger oder alkoholischer oder ammoniakalischer Lösung imprägniert. Sowohl an einen Ionenaustausch als auch an eine Imprägnierung schließt sich zumindest eine Trocknung und wahlweise eine abermalige Calcination an.

Im einzelnen verfährt man z.B. so, daß man Wolframsäure ($H_2WO_4$) oder $Ce(NO_3)_3 \times 6H_2O$ in Wasser löst. Mit dieser Lösung wird dann der verstrangte oder unverstrangte Zeolith eine gewisse Zeit, ca.

30 min., getränkt. Die überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcination bei ca. 500°C kann das so gewonnene zeolithische Material mit und ohne Bindemittel zu Strängen oder Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder mit Wasserdampf unterwirft. Dabei geht man vorteilhaft z.B. so vor, daß man das zeolithische Pulver vor seiner Verformung mit 0,001 n bis 2 n, bevorzugt 0,05 n bis 0,5 n Flußsäure 1 bis 3 h unter Rückfluß behandelt. Nach Abfiltrieren und Auswaschen wird bei 100 bis 160°C getrocknet und bei 400 bis 550°C calciniert. Auch eine Behandlung der Zeolithe nach ihrer Verformung mit Bindemittel mit Salzsäure kann zweckmäßig sein. Hierbei wird der Zeolith z.B. 1 bis 3 h zwischen 60 und 80°C mit einer 3 bis 25 %igen Salzsäure behandelt, anschließend ausgewaschen, bei 100 bis 160°C getrocknet und bei 400 bis 550°C calciniert. Man kann den Zoelithen auch durch Aufbringen von Phosphorverbindungen, wie Trimethoxyphosphat, modifizieren.

Nach einer eventuellen Desaktivierung der zeolithischen Katalysatoren, die beim Verfahren der Erfindung durch Koksabscheidung eintreten kann, lassen sich diese durch Abbrennen der Koksablagerung mit Luft oder mit einem Luft/$N_2$-Gemisch bei 400 bis 550°C, bevorzugt 500°C, in einfacher Weise regenerieren, wodurch sie ihre Anfangsaktivität zurückerhalten.

Man kann auch durch eine partielle Verkokung (pre-coke) die Aktivität des Katalysators für ein Selektivitätsoptimum des gewünschten Reaktionsproduktes einstellen.

Im allgemeinen werden die Katalysatoren wahlweise als 2 bis 4 mm Stränge, als Tabletten mit 3 bis 5 mm Durchmesser, als Pulver mit Teilchengrößen von 0,3 bis 0,5 mm oder (als Wirbelkontakt) von 0,1 bis 0,5 mm eingesetzt.

Die Isomerisierung bzw. Dehydratisierung der Aldehyde an den Zeolithen läßt sich in der Flüssigphase z.B. bei Temperaturen von 30 bis 300°C durchführen. Vorzugsweise wird die Umsetzung in der Gasphase bei Temperaturen von 100 bis 600°C, insbesondere bei 250 bis 500°C, vorgenommen. Die Belastung (WHSV) beträgt 0.1 bis 20, vorzugsweise 0.5 bis 5 kg Gemisch pro kg Katalysator und Stunde. Das Verfahren kann chargenweise oder kontinuierlich, drucklos oder unter Druck, z.B. in einem Strömungsreaktor oder Wirbelreaktor, durchgeführt werden.

Das Verfahrensfließbild ist in der Zeichnung wiedergegeben. Hierbei wird das Ausgangsgemisch über Leitung 1 in den Reaktor 2 eingespeist. Die Reaktionsprodukte werden über Leitung 3 in die erste Destillationskolonne 4 geleitet, in der über Kopf (Leitung 5) Isopren und gegebenenfalls Isobuten abgetrennt wird. Die Sumpfprodukte werden über Leitung 6 in die zweite Destillationskolonne 7 gegeben. Hierbei wird das Methylisopropylketon von den restlichen Produkten abgetrennt und über Leitung 8 gewonnen. Die Sumpfprodukte der zweiten Destillation können über Leitung 9 zurückgeführt oder über Leitung 10 entsorgt werden.

Methylisopropylketon wird vorwiegend als Zwischenprodukt zur Herstellung von Pharmazeutika, Herbiziden und indigoiden Farbstoffen (Fischer-Synthese) verwendet, während Isopren zur Herstellung von cis-1,4-Polyisopren dient. Weitere Verwendungsmöglichkeiten sind in Ullmanns Encyclopädie der technischen Chemie 4. Auflage (1977), Bd. 14, S. 198 und Bd. 13, S. 385 und 386 beschrieben.

## Beispiele

Der verwendete Katalysator wurde in einer hydrothermalen Synthese aus 640 g $SiO_2$ (hochdisperse Kieselsäure), 121 g $H_3BO_3$, 8000 g einer wäßrigen Hexandiamin-Lösung (Mischung 50:50 Gew.%) bei 170°C unter autogenem Druck in einem Rührautoklaven hergestellt. Nach Abfiltrieren und Auswaschen wurde das kristalline Reaktionsprodukt bei 100°C/24 h getrocknet und bei 500°C/24 h calciniert. Es wurde ein Borosilikatzeolith vom Pentasiltyp erhalten, der 94,2 Gew.% $SiO_2$ und 2,3 Gew.% $B_2O_3$ enthielt. Aus diesem Zeolithen wurden 2 mm-Stränge hergestellt, die bei 100°C getrocknet und bei 500°C/24 h calciniert wurde.

Die Versuche wurden in einem Rohrreaktor mit 100 ml Katalysatorfüllvolumen und 2 cm Innendurchmesser unter isothermen Bedingungen durchgeführt.

Die Thermostatisierung des Reaktors erfolgt in einem Salzbad. Das Umsetzungsgemisch wurde in einem vorgeschalteten Verdampfer bei 120°C verdampft und gasförmig bei Temperaturen von 400 bis 450°C und einer Belastung WHSV = 1 - 2 $h^{-1}$ über den Zeolith-Katalysator geleitet. Die Reaktionsprodukte wurden kondensiert und in Kühlfallen aufgefangen. Die Identifizierung und quantitative Bestimmung der Reaktionsprodukte erfolgte nach üblichen Methoden, z.B. durch Destillation, Gaschromatographie (GC) oder GC-MS-Kopplung.

Beispiele 1 bis 3

Ausgehend von einem Einsatzgemisch, das 75,8 Gew.% Pivalinaldehyd und 17,5 Gew.% tert.-Butanol sowie zusätzlich 3,5 Gew.% Isovaleraldehyd und 3,2 Gew.% $C_8$-Kohlenwasserstoffe enthielt, wurden die in Tabelle 1 zusammengefaßten Ergebnisse erhalten.

Tabelle 1

| Temperatur [°C] | 400 | 450 | 450 |
|---|---|---|---|
| WHSV [$h^{-1}$] | 1 | 1 | 2 |
| Umsatz Pivalinaldehyd in % | 96,2 | 95,3 | 96,1 |
| Selektivität[1] % | | | |
| Methylisopropylketon | 79,5 | 60,9 | 68,7 |
| Isopren | 18,7 | 36,7 | 27,2 |
| Gesamtselektivität | 98,2 | 97,6 | 95,9 |

[1] bezogen auf umgesetzten Pivalinaldehyd

Das tert.-Butanol wurde bei allen 3 Versuchen zu Isobuten mit 85 bis 90 % Selektivität umgesetzt.

Beispiele 4 bis 7

Zu den Beispielen 4 bis 7 wurde ein Reaktionsgemisch aus 48,5 Gew.% Pivalinaldehyd, 13,2 Gew.% 3-Methylbutanal, 20,0 Gew.% tert.-Butanol, 13,4 Gew.% n-Butyraldehyd, Rest Trimethylpentan, Dimethylhexan und Wasser eingesetzt. Die hiermit erzielten Versuchsergebnisse sind in Tabelle 2 zusammengefaßt.

Tabelle 2

| Temperatur [°C] | 400 | 450 | 400 | 450 |
|---|---|---|---|---|
| WHSV [$h^{-1}$] | 1 | 1 | 2 | 2 |
| Umsatz in % | | | | |
| Pivalinaldehyd | 96,3 | 96,3 | 96,2 | 96,3 |
| 3-Methylbutanal | 45,9 | 58,2 | 22,7 | 44,4 |
| Pentanale | 85,5 | 88,2 | 80,5 | 85,2 |
| Selektivität % | | | | |
| Methylisopropylketon[1] | 72,9 | 60,7 | 78,9 | 66,9 |
| Isopren[2] | 22,5 | 31,1 | 16,9 | 26,6 |
| Gesamtselektivität | 95,4 | 91,8 | 95,8 | 93,5 |

[1] bezogen auf umgesetzten Pivalinaldehyd
[2] bezogen auf umgesetzte Pentanale

Das im Einsatzgemisch enthaltene tert.-Butanol wurde bei 100 % Umsatz mit 85 bis 88 %iger Selektivität zu Isobuten dehydratisiert.

Beispiel 8

In Beispiel 8 wird ein Langzeitversuch wiedergegeben, der mit dem gleichen Reaktionsgemisch wie in den Beispielen 4 bis 7 durchgeführt wurde. Als Versuchsbedingungen wurden 400°C und WHSV = 2 $h^{-1}$ gewählt. Tabelle 3 gibt die Versuchsergebnisse von 4 Bilanzzeiträumen wieder.

Tabelle 3

| Bilanzzeitraum | 1 | 2 | 3 | 4 |
|---|---|---|---|---|
| Umsatz in % | | | | |
| Pivalinaldehyd | 96,9 | 97,0 | 97,0 | 97,0 |
| Isovaleraldehyd | 33,9 | 33,3 | 24,5 | 24,8 |
| Σ Penanale | 83,4 | 83,4 | 81,5 | 81,6 |
| Selektivität % | | | | |
| Methylisopropylketon[1] | 77,6 | 76,6 | 76,2 | 76,0 |
| Isopren[2] | 19,3 | 21,0 | 21,2 | 21,0 |
| Gesamtselektivität | 96,9 | 97,6 | 97,4 | 97,0 |
| Laufzeit h | 23 | 24 | 24 | 29 |

[1] bezogen auf umgesetzten Pivalinaldehyd
[2] bezogen auf umgesetzte Pentanale

Das im Einsatzgemisch enthaltene tert.-Butanol wurde bei 100 % Umsatz mit 85 bis 90 %iger Selektivität zu Isobuten umgesetzt.

**Patentansprüche**

1. Verfahren zur Herstellung von Methylisopropylketon und Isopren durch Umsetzung von Pivalinaldehyd an zeolithischen Katalysatoren bei Temperaturen von 30 bis 600°C, dadurch gekennzeichnet, daß man für die Umsetzung Hydroformylierungsgemische des Isobutens oder isobutenhaltiger $C_4$-Schnitte verwendet.
2. Verfahren nach Anspruch 1, dadurch gekennzeichnet, daß man die Hydroformylierungsprodukte des Isobutens oder isobutenhaltiger $C_4$-Schnitte nach vollständiger oder teilweiser Entfernung des 3-Methylbutanals umsetzt.
3. Verfahren nach Anspruch 1, dadurch gekennzeichnet, daß man die Umsetzung in der Gasphase bei Temperaturen von 100 bis 600°C vornimmt.

**Claims**

1. A process for the preparation of methyl isopropyl ketone and isoprene by converting pivalaldehyde over a zeolite catalyst at from 30 to 600°C, wherein a hydroformylation mixture of isobutene or of an isobutene-containing $C_4$ cut is used for the conversion.
2. A process as claimed in claim 1, wherein the hydroformylation products of isobutene or of an isobutene-containing $C_4$ cut are converted after complete or partial removal of the 3-methylbutanal.
3. A process as claimed in claim 1, wherein the conversion is carried out in the gas phase at from 100 to 600°C.

**Revendications**

1. Procédé de préparation de méthylisopropylcétone et d'isoprène par réaction de pivalinaldéhyde sur des catalyseurs zéolithiques et à températures élevées, caractérisé en ce qu'on utilise pour la réaction des mélanges d'hydroformylation de l'isobutène ou d'une coupe en $C_4$ contenant de l'isobutène.
2. Procédé selon la revendication 1, caractérisé en ce qu'on fait réagir les produits d'hydroformylation de l'isobutène ou d'une coupe en $C_4$ contenant de l'isobutène après élimination totale ou partielle du 3-méthylbutanal.
3. Procédé selon la revendication 1, caractérisé en ce qu'on effectue la réaction en phase gazeuse à des températures de 100 à 600°C.

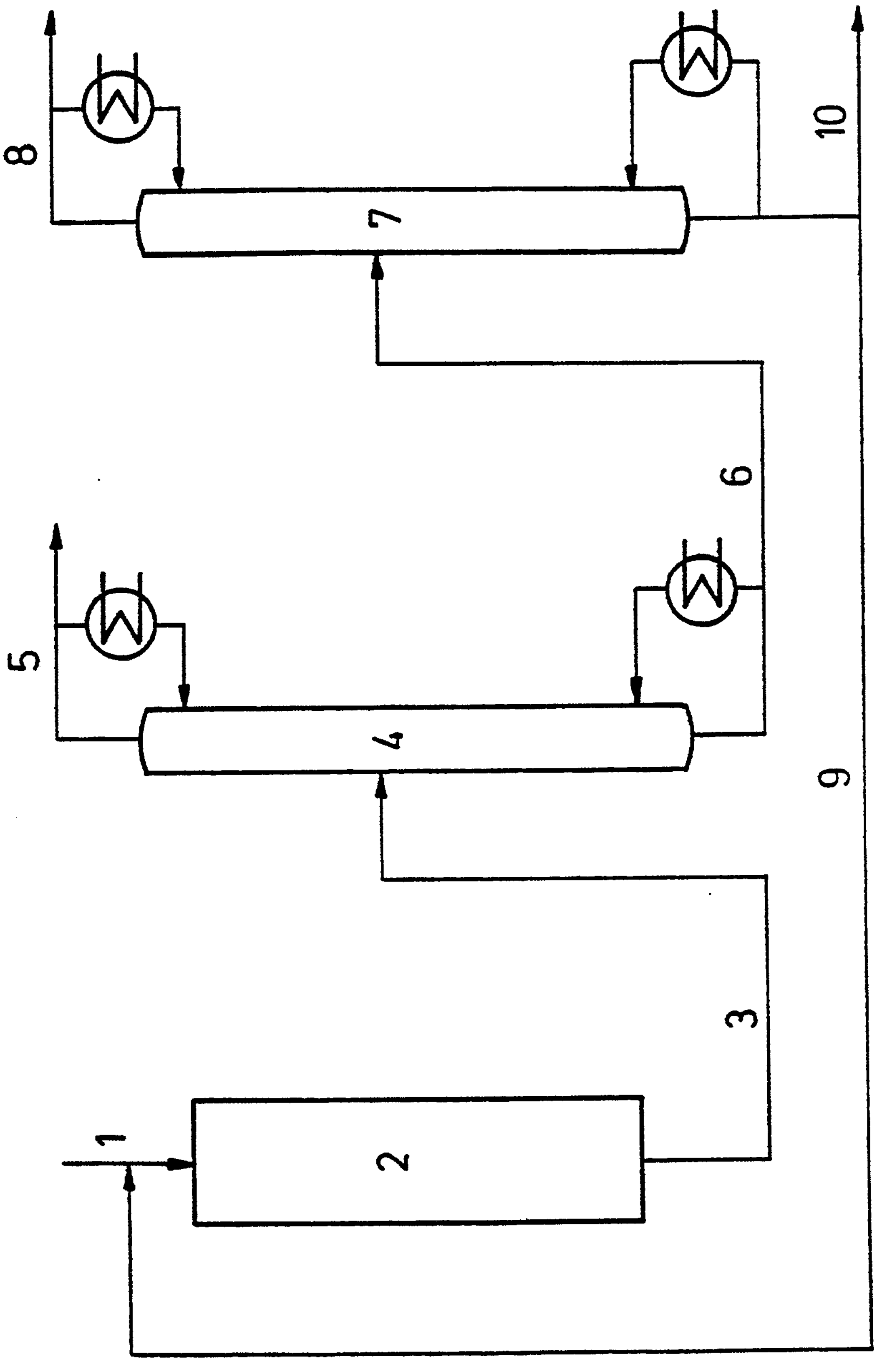
1
2
3
4
5
6
7
8
9
10